(19) Europäisches Patentamt
European Patent Office
Office européen des brevets

(11) **EP 4 672 251 A1**

(12) # EUROPEAN PATENT APPLICATION

(43) Date of publication:
**31.12.2025 Bulletin 2026/01**

(21) Application number: **25184145.8**

(22) Date of filing: **20.06.2025**

(51) International Patent Classification (IPC):
**G16C 60/00** (2019.01)     **C09D 175/04** (2006.01)

(52) Cooperative Patent Classification (CPC):
**G16C 60/00; C09D 175/04; G06F 30/20;**
**G16C 20/10; G16C 20/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **25.06.2024 IN 202421048797**

(71) Applicant: **Tata Consultancy Services Limited
Mumbai, Maharashtra 400 021 (IN)**

(72) Inventors:
• **GUPTA, Ambesh
411057 Pune, Maharashtra (IN)**

• **MAITI, Soumyadipta
411057 Pune, Maharashtra (IN)**
• **SAINI, Parvesh
411057 Pune, Maharashtra (IN)**
• **SHRIVASTAVA, Surabhi
411057 Pune, Maharashtra (IN)**
• **KAUSLEY, Shankar Balajirao
411057 Pune, Maharashtra (IN)**
• **RAI, Beena
411057 Pune, Maharashtra (IN)**

(74) Representative: **Goddar, Heinz J.
Boehmert & Boehmert
Anwaltspartnerschaft mbB
Pettenkoferstrasse 22
80336 München (DE)**

(54) **COMBINED PHYSICAL AND CHEMICAL MODELS FOR ESTIMATING SERVICE LIFETIME AND DEGRADATION OF COATING MATERIALS**

(57) Coating materials such as paints, and/or coatings used to protect and improve the aesthetics of a material surface, develop the problem of degradation under environmental exposure. The degradation causes the coating to fail unexpectedly in its required applications. To keep this in check, the service life and change in material properties of a paint/coating need to be predicted before its use. Present disclosure provides system and method that implement a combined model to estimate the service lifetime and predict the chemical and physical changes in a coating material under various weathering conditions. The combined model captures the chemical modifications and physical modifications and affected properties like surface roughness, thickness loss, fracture toughness, gloss loss respectively. Chemical changes and physical changes are estimated correlated to help in estimating the service lifetime and degradation of the coating material.

receiving, via one or more hardware processors, a plurality of inputs pertaining to a coating material comprising a thickness, a polymer chemistry, a sunlight-Ultraviolet (UV) time series, an oxygen concentration, a moisture concentration, one or more polymer concentration profiles, and a product concentration of polymers — 202

generating, via the one or more hardware processors, (i) a first model and a second model based on the plurality of inputs, wherein the second model is further based on one or more oxygen concentration profiles and number of reactive photons obtained from the first model — 204

combining, via the one or more hardware processors, the first model and the second model to obtain a combined model — 206

estimating, via the one or more hardware processors, a service lifetime, and a degradation of the coating material using the combined model — 208

**FIG. 2**

EP 4 672 251 A1

**Description**

CROSS-REFERENCE TO RELATED APPLICATIONS AND PRIORITY

The present application claims priority to Indian application no. 202421048797 filed on June 25, 2024.

TECHNICAL FIELD

The disclosure herein generally relates to coating materials, and, more particularly, to combined physical and chemical models based estimation of service lifetime and degradation of coating materials.

BACKGROUND

Coating materials such as paints, and/or coatings used to protect and improve the aesthetics of a material surface, develop the problem of degradation under environmental exposure. The degradation causes the coating to fail unexpectedly in its required applications. To keep this in check, the service life and change in material properties of a paint/coating need to be predicted before its use. Estimating change in properties requires time enduring and repeated testing. Such approaches lead to more costs with respect to architectural, industrial, functional and transportational coatings, etc.

SUMMARY

Embodiments of the present disclosure present technological improvements as solutions to one or more of the above-mentioned technical problems recognized by the inventors in conventional systems.

For example, in one aspect, there is provided a processor implemented method for combined physical and chemical models based estimation of service lifetime and degradation of coating materials. The method comprises receiving, via one or more hardware processors, a plurality of inputs pertaining to a coating material comprising a thickness, a polymer chemistry, a sunlight-Ultraviolet (UV) time series, an oxygen concentration, a moisture concentration, one or more polymer concentration profiles, and a product concentration of polymers; generating, via the one or more hardware processors, (i) a first model and a second model based on the plurality of inputs, wherein the second model is further based on one or more oxygen concentration profiles and number of reactive photons obtained from the first model; combining, via the one or more hardware processors, the first model and the second model to obtain a combined model; and estimating, via the one or more hardware processors, a service lifetime, and a degradation of the coating material using the combined model.

In an embodiment, the first model is obtained by generating a first set of parameters using the plurality of inputs; iteratively performing: applying, by using a first solver, a first set of governing equations on the first set of parameters to obtain a second set of parameters; and applying, by using a second solver, a second set of governing equations on the second set of parameters, until an optimized set of parameters is obtained; and generating the first model using the optimized set of parameters.

In an embodiment, the second model is obtained by performing a Monte-Carlo (MC) simulation technique on the optimized set of parameters and the plurality of inputs to obtain a set of simulation parameters; estimating a pixel damage location, and a MC event time series from the set of simulation parameters; estimating a pore damage time, and a pixel spatiotemporal damage status using the pixel damage location, and the MC event time series; obtaining a first updated pixel spatiotemporal damage region based on an associated pit formation and growth identified in the pore damage time, and the pixel spatiotemporal damage status; and filtering one or more unconnected islands from the first updated pixel spatiotemporal damage region to obtain a second updated pixel spatiotemporal damage region, wherein the second updated pixel spatiotemporal damage region serves as the second model.

In an embodiment, the combined model is obtained by correlating each of the one or more polymer concentration profiles of the first model with one or more timesteps of the second updated pixel spatiotemporal damage region serving as the second model to obtain the combined model, wherein the combined model comprises the one or more polymer concentration profiles from the first model, and a time scaling factor and the second updated pixel spatiotemporal damage region serving as the second model.

In an embodiment, the step of estimating the service lifetime, and the degradation of the coating material using the combined model comprises: developing a surface profile of the coating material using the pixel spatiotemporal damage status associated with the second model; calculating a thickness loss and a roughness parameter of the coating material based on the surface profile; calculating a gloss loss, a fracture toughness and a contact wetting angle of the coating material using the thickness loss and the roughness parameter; and estimating the service lifetime, and the degradation of the coating material based on the gloss loss, the fracture toughness, and the contact wetting angle.

In another aspect, there is provided a processor implemented system for combined physical and chemical models based estimation of service lifetime and degradation of coating materials. The system comprises: a memory storing instructions; one or more communication interfaces; and one or more hardware processors coupled to the memory via the one or more communication interfaces, wherein the one or more hardware processors are configured by the instructions to: receive a plurality of inputs pertaining to a coating material comprising a thickness, a polymer chemistry, a sunlight-Ultraviolet (UV) time series, an oxygen concentration, a moisture concentration, one or more polymer concentration profiles, and a product concentration of polymers; generate (i) a first model and a second model based on the plurality of inputs, wherein the second model is further based on one or more oxygen concentration profiles and number of reactive photons obtained from the first model; combine the first model and the second model to obtain a combined model; and estimate a service lifetime, and a degradation of the coating material using the combined model.

In an embodiment, the first model is obtained by generating a first set of parameters using the plurality of inputs; iteratively performing: applying, by using a first solver, a first set of governing equations on the first set of parameters to obtain a second set of parameters; and applying, by using a second solver, a second set of governing equations on the second set of parameters, until an optimized set of parameters is obtained; and generating the first model using the optimized set of parameters.

In an embodiment, the second model is obtained by performing a Monte-Carlo (MC) simulation technique on the optimized set of parameters and the plurality of inputs to obtain a set of simulation parameters; estimating a pixel damage location, and a MC event time series from the set of simulation parameters; estimating a pore damage time, and a pixel spatiotemporal damage status using the pixel damage location, and the MC event time series; obtaining a first updated pixel spatiotemporal damage region based on an associated pit formation and growth identified in the pore damage time, and the pixel spatiotemporal damage status; and filtering one or more unconnected islands from the first updated pixel spatiotemporal damage region to obtain a second updated pixel spatiotemporal damage region, wherein the second updated pixel spatiotemporal damage region serves as the second model.

In an embodiment, the combined model is obtained by correlating each of the one or more polymer concentration profiles of the first model with one or more timesteps of the second updated pixel spatiotemporal damage region serving as the second model to obtain the combined model, and wherein the combined model comprises the one or more polymer concentration profiles from the first model, and a time scaling factor and the second updated pixel spatiotemporal damage region serving as the second model.

In an embodiment, the service lifetime, and the degradation of the coating material are estimated using the combined model by developing a surface profile of the coating material using the pixel spatiotemporal damage status associated with the second model; calculating a thickness loss and a roughness parameter of the coating material based on the surface profile; calculating a gloss loss, a fracture toughness and a contact wetting angle of the coating material using the thickness loss and the roughness parameter; and estimating the service lifetime, and the degradation of the coating material based on the gloss loss, the fracture toughness, and the contact wetting angle.

In yet another aspect, there are provided one or more non-transitory machine-readable information storage mediums comprising one or more instructions which when executed by one or more hardware processors cause implementing combined physical and chemical models based estimation service lifetime and degradation of coating materials by receiving a plurality of inputs pertaining to a coating material comprising a thickness, a polymer chemistry, a sunlight-Ultraviolet (UV) time series, an oxygen concentration, a moisture concentration, one or more polymer concentration profiles, and a product concentration of polymers; generating (i) a first model and a second model based on the plurality of inputs, wherein the second model is further based on one or more oxygen concentration profiles and number of reactive photons obtained from the first model; combining the first model and the second model to obtain a combined model; and estimating a service lifetime, and a degradation of the coating material using the combined model.

In an embodiment, the first model is obtained by generating a first set of parameters using the plurality of inputs; iteratively performing: applying, by using a first solver, a first set of governing equations on the first set of parameters to obtain a second set of parameters; and applying, by using a second solver, a second set of governing equations on the second set of parameters, until an optimized set of parameters is obtained; and generating the first model using the optimized set of parameters.

In an embodiment, the second model is obtained by performing a Monte-Carlo (MC) simulation technique on the optimized set of parameters and the plurality of inputs to obtain a set of simulation parameters; estimating a pixel damage location, and a MC event time series from the set of simulation parameters; estimating a pore damage time, and a pixel spatiotemporal damage status using the pixel damage location, and the MC event time series; obtaining a first updated pixel spatiotemporal damage region based on an associated pit formation and growth identified in the pore damage time, and the pixel spatiotemporal damage status; and filtering one or more unconnected islands from the first updated pixel spatiotemporal damage region to obtain a second updated pixel spatiotemporal damage region, wherein the second updated pixel spatiotemporal damage region serves as the second model.

In an embodiment, the combined model is obtained by correlating each of the one or more polymer concentration profiles of the first model with one or more timesteps of the second updated pixel spatiotemporal damage region serving as the second model to obtain the combined model, and wherein the combined model comprises the one or more polymer concentration profiles from the first model, and a time scaling factor and the second updated pixel spatiotemporal damage region serving as the second model.

In an embodiment, the step of estimating the service lifetime, and the degradation of the coating material using the combined model comprises: developing a surface profile of the coating material using the pixel spatiotemporal damage status associated with the second model; calculating a thickness loss and a roughness parameter of the coating material based on the surface profile; calculating a gloss loss, a fracture toughness and a contact wetting angle of the coating material using the thickness loss and the roughness parameter; and estimating the service lifetime, and the degradation of the coating material based on the gloss loss, the fracture toughness, and the contact wetting angle.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 depicts an exemplary system that implements a combined physical and chemical model for estimating service lifetime and degradation of coating materials, in accordance with an embodiment of the present disclosure.

FIG. 2 depicts an exemplary flow chart illustrating a method that implements a combined physical and chemical model for estimating service lifetime and degradation of coating materials, using the system of FIG. 1, in accordance with an embodiment of the present disclosure.

FIG. 3 depicts discretization of space along the depth direction in the coating material, in accordance with an embodiment of the present disclosure.

FIG. 4A depicts a graphical representation illustrating a comparison of polymer normalized concentration from initial solution and experiment, in accordance with an embodiment of the present disclosure.

FIG. 4B depicts a graphical representation illustrating a comparison of carboxylic acid normalized concentration from initial solution and experiment, in accordance with an embodiment of the present disclosure.

FIG. 5 depicts a flow diagram illustrating the method for estimating the second set of parameters for generating the second model, in accordance with an embodiment of the present disclosure.

FIGS. 6A through 6D depict graphical representations illustrating concentrations profile matching from model and experiments, wherein FIGS. 6A and 6B depict middle of iterations, and FIGS. 6C and 6D depict from the final iteration, in accordance with an embodiment of the present disclosure.

FIG. 7 depicts a graphical representation illustrating averaged concentration profiles from a first solver (e.g., a partial differential equation (PDE) solver) during the optimization at different iterations from a second solver (e.g., an ordinary differential equation (ODE) solver), in accordance with an embodiment of the present disclosure.

FIGS. 8A through 8C depict graphical representations illustrating concentration profiles of components from a second model using an optimized set of kinetics parameters at different depths, in accordance with an embodiment of the present disclosure.

FIG. 9 depicts the coating material as simulation box of specific depth divided into cells of specific dimension, in accordance with an embodiment of the present disclosure.

FIGS. 10A and 10B depict surface topography along the depth of the coating by the Monte Carlo simulations at MC time of degraded events (e.g., 86888 events in FIG. 10A, 445707 events in FIG. 10B), in accordance with an embodiment of the present disclosure.

FIGS. 11A and 11B show the surface topography at two timesteps of subsequent successful coating degradation (of dimensions $20 \mu m$ x $20 \mu m$ x 40nm) in which vacuum/pit is represented by the black color, undegraded polymer is represented by light grey color, pore is represented by dark grey color, in accordance with an embodiment of the present disclosure.

FIG. 12 depicts a graphical representation illustrating a comparison of a normalized RH (polymer) concentration between chemical kinetics model and physical degradation model for a 70-micron coating, in accordance with an embodiment of the present disclosure.

FIG. 13 depicts a graphical representation illustrating a comparison of the normalized RH (polymer) concentration between chemical kinetics model and physical degradation model for a 20-micron depth coating, in accordance with an embodiment of the present disclosure.

FIGS. 14A through 14D depict graphical representations illustrating surface roughness profiles developed from the pixel temporal damage status/region (e.g., the first and second updated pixel spatiotemporal damage region) of a 70-micron depth coating at various times of 17.57 h, 200 h, 400 h, and 800 h, respectively, in accordance with an embodiment of the present disclosure.

FIGS. 15A and 15B depict graphical representations illustrating thickness of the coating varying with time depicting the loss with real accelerated weathering time from physical model (e.g., refer FIG. 15A), and with natural outdoor exposure of epoxy-based PU coating (e.g., refer FIG. 15B), in accordance with an embodiment of the present disclosure.

FIG. 16 depicts a graphical representation illustrating a root mean square (RMS) Roughness of the 70-micron depth coating with accelerated weathering degradation time evaluated from the Monte Carlo simulations, in accordance with an embodiment of the present disclosure.

FIG. 17 depicts a graphical representation illustrating a root mean square (RMS) Roughness of the acrylic based polyurethane coating from weathering under different accelerated exposure conditions, in accordance with an embodiment of the present disclosure.

FIG. 18 depicts a graphical representation illustrating a change in relative fracture toughness of the coating material with time of degradation, in accordance with an embodiment of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Paints and/or coatings (also referred to as coating materials) have been widely used in industries on materials, automobiles, civil structures to improve surface finish, provide protection from environmental exposure, provide gloss, etc. These coating materials are used specifically for these purposes and thus they act as a functional material. For the coating functional materials, a high service lifetime is desired. Knowledge of gradual time dependent failure of the coatings or the changes it undergoes during its lifetime becomes essential in application of coatings. Paints used in industries undergo a high amount of testing of weathering. For example, Florida natural exposure testing is done for 5-10 years to estimate the service lifetime of paints. These tests help in understanding the changes in the characteristics of the paint coatings. As these tests take place for an exceptionally long time, accelerated testing in weathering chambers is conducted in recent times taking around 5-8 months of time. However, a mathematical simulation of these weathering of paint coatings can only take significantly shorter time like around few days to few weeks to mimic such experimental natural and accelerated testing. Chemical modifications and physical modifications to the coatings affect the service lifetime. It may be possible that coating does not fulfill the requirements in sense of its physical modifications while chemically it does. Thus, simulating the physical modifications due to external weathering becomes important.

In the present disclosure, systems and methods described herein have considered an automotive coating for estimating degradation under ultraviolet (UV) exposure only to mimic an accelerated weathering testing. Chemistry of the paint coating and its degradation mechanisms under given exposure were identified from the literature. Chemical model based on the kinetics of the reactions involved in degradation was developed and optimized with the available experimental data. Chemical modifications in terms of concentrations of components were obtained as output of this model. Also, a model based on Monte Carlo simulations has been developed to predict the Physical behavior of the coating surface under exposure with time. Surface topography and statistical surface attributes like Roughness, Gloss loss are then estimated using the developed model. Mechanical properties like impact strength, mass loss, etc. can also be estimated. All these surface modifications can be translated to the chemical model as the physical model is related successfully to the chemical model in a concurrent timewise manner (e.g., a combined model comprising the chemical kinetics model and a physical degradation model). These properties function as a good measure for estimation of service lifetime in terms of physical and chemical modifications.

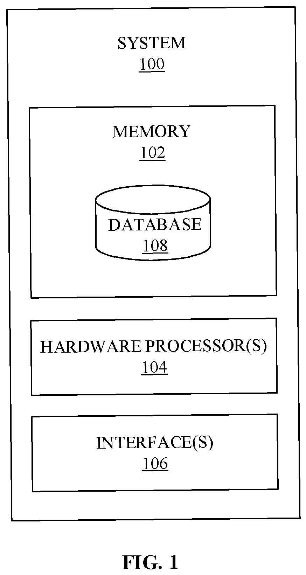

Referring now to the drawings, and more particularly to FIGS. 1 through 18, where similar reference characters denote corresponding features consistently throughout the figures, there are shown preferred embodiments, and these embodiments are described in the context of the following exemplary system and/or method.

FIG. 1 depicts an exemplary system 100 that implements a combined physical and chemical model for estimating service lifetime and degradation of coating materials, in accordance with an embodiment of the present disclosure. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is/are configured to fetch and execute computer-readable instructions stored in the memory. In an embodiment, the system 100 can be implemented in a variety of computing systems, such as laptop computers, notebooks, hand-held devices (e.g., smartphones, tablet phones, mobile communication devices, and the like), workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random-access memory (SRAM) and dynamic-random access memory (DRAM), and/or non-volatile memory, such as read only memory (ROM), erasable programmable ROM, flash memories, hard disks, optical disks, and magnetic tapes. In an embodiment, a database 108 is comprised in the memory 102, wherein the database 108 comprises information various inputs pertaining to one or more coating materials. The database 108 further comprises various models (e.g., say chemical kinetics model, physical degradation model, and the like) which are generated using the various inputs, and the like. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

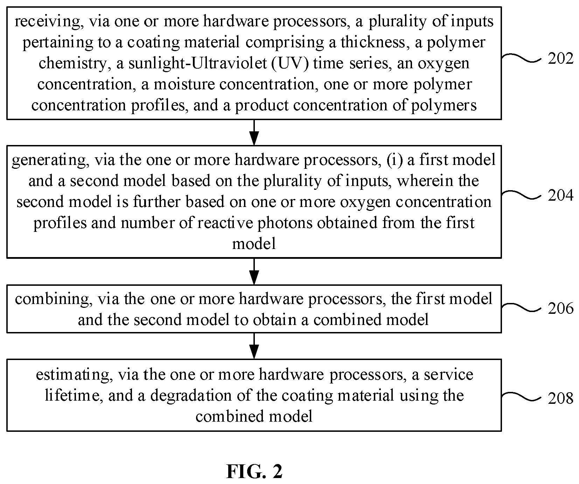

FIG. 2, with reference to FIG. 1, depicts an exemplary flow chart illustrating a method that implements a combined physical and chemical model for estimating service lifetime and degradation of coating materials, using the system 100 of FIG. 1, in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to components of the system 100 of FIG. 1, the flow diagram as depicted in FIG. 2, and FIGS. 3 through 18.

At step 202 of the method of the present disclosure, the one or more hardware processors 104 receive a plurality of inputs pertaining to a coating material comprising a thickness, a polymer chemistry, a sunlight-Ultraviolet (UV) time series, an oxygen concentration, a moisture concentration, one or more polymer concentration profiles, and a product concentration of polymers. In the present disclosure, the system 100 and the method have considered an automotive coating for estimating degradation under UV exposure only to mimic an accelerated weathering testing. Chemistry of the paint coating and its degradation mechanisms under given exposure were identified from the literature. Chemical model based on the kinetics of the reactions involved in degradation was developed and optimized with an available experimental data (not shown in FIGS.). Chemical modifications in terms of concentrations of components were obtained as output of this model. Also, a model based on Monte Carlo simulations has been developed to predict the Physical behavior of the coating surface under exposure with time. Surface topography and statistical surface attributes like Roughness, Gloss loss can be estimated using the developed model. Mechanical properties like impact strength, mass loss, etc. can also be estimated. All these surface modifications can be translated to the chemical model as the physical model is related successfully to the chemical model in a concurrent timewise manner. These properties function as a good measure for estimation of service lifetime in terms of physical and chemical modifications. Paint coating contains polymer and binder which degrades under the environmental exposure and defines the lifetime of a coating. For automotive coating selected, the major component was acrylic based polyurethane. This polymer was chosen as this is one of the most widely used polymer and binder in both clear coat and base coat. Coating thickness was taken to be in the range of 60 - 100 $\mu$m. The system 100 has selected accelerated testing environmental conditions which involve UV - a light exposure without the presence of moisture. The typical light intensity in this accelerated testing environment is around 30 W/m$^2$.

At step 204 of the method of the present disclosure, the one or more hardware processors 104 generate (i) a first model and a second model based on the plurality of inputs. The second model is further based on one or more oxygen concentration profiles and number of reactive photons obtained from the first model. The first model is referred to as 'a chemical kinetics model' and may be interchangeably used herein. The second model is referred to as 'a physical degradation model' and may be interchangeably used herein.

The first model (e.g., the chemical kinetics model) is obtained by generating a first set of parameters using the plurality of inputs. For instance, the first set of parameters include reactions, approximate reaction rates, mass balance stoichiometry, and initial species concentrations pertaining to the coating material received in step 202. Further, the first set of parameters serves as an input to an iterative model which implements a plurality of solvers to obtain an optimized set of parameters. In the present disclosure, the iteration includes applying a first set of governing equations on the first set of parameters to obtain a second set of parameters by using a first solver (e.g., a partial differential equation (PDE) solver). The second set of parameters includes, but is not limited to concentration vs time profiles, reactive photon counts, and the like. The iteration further includes applying a second set of governing equations on the second set of parameters by using a second solver (e.g., ordinary differential equation solver). Both of the solvers are applied to the first set of parameters and the second set of parameters respectively until the optimized set of parameters is obtained. The optimized set of parameters includes, but is not limited to, optimized reaction rate parameters. Using the optimized set of parameters (or the optimized reaction rate parameters), the system 100 generates the chemical kinetics model/first model.

The above step of generating the first model may be better understood by way of following description. More specifically, the below description illustrates the step of generating the first set of parameters using the plurality of inputs.

Acrylic based polyurethane degrades under UV exposure by the photodegradation mechanism as identified from the polymer database. The set of reactions involved in mechanism is as follows.

$$X - X + h\vartheta \rightarrow 2X^* \quad\text{............................} \quad (R1)$$

$$R1 - O - CO - NH - CH_2 - R2 + X^* \rightarrow R^* + XH \quad\text{......................} (R2)$$

$$R1 - OCO - NH - CH^* - R2 + O_2 \rightarrow R1 - O - CO - NH - C - (O - O^*) - R2 \quad\text{............} . (R3)$$

$$2 R1 - O - CO - NH - C - (O - O^*) - R2 \rightarrow R1 - O - CO - NH - C - (OH) - R2 + R1 - O - CO - NH - C-= (0) - R \quad\text{..................} (R4)$$

$$R1 - O - CO - NH - C - (O - O^*) - R2 + R_1 - O - CO - NH - CH_2 - R2 \rightarrow R2COOH + R1 - O - CO - NH_2 + R1 - O - CO - NH - CH^* - R2 \quad\text{................} (R5)$$

$$2 R1 - O - CO - NH - CH^* - R2 \rightarrow R - R \quad\text{....................} . (R6)$$

The first reaction is the formation of free radical from the impurity/ chromophores present in the coating. Impurity free radical attacks on the polymer component and produces a polymer radical (reaction R2). This polymer radical reacts with the oxygen present and generates a per-oxy free radical (reaction R3). This per-oxy free radical reacts with itself (reaction R4) or propagates the polymer degradation by attacking on polymer and producing more polymer radicals. These polymer radicals further take place into reaction as shown in the reactions R3, and R5 and therefore increasing the degradation rate. Also, some polymer free radicals can react with themselves to form a cross linked polymer which terminates the degradation reaction (refer R6). Thus, the above defined mechanism completes the degradation reactions of polyurethane type polymer.

Oxygen in these reactions is available from the atmosphere and it diffuses into the coating from the surface. Thus, diffusion effects on the reactions of coating becomes important too. Therefore, there are in total seven reactions in coating degradation. For all the components involved, the initial concentrations and boundary conditions are taken for the coating chemistry are shown in Table 1.

Table 1: Components involved in the degradation reactions along with their concentration expressions, initial concentrations, and boundary conditions.

| S.No. | Species | Concentration expression | Initial concentrations and boundary conditions |
|---|---|---|---|
| 1 | Main polymer (R1-COONHCH$_2$-R2) | $[RH]$ | Initial Cross-linking density |
| 2 | Impurity (X-X or X-H) | $[X - X]$ | 1% of initial cross-linking density |
| 3 | Impurity radical (X*) | $[X^*]$ | 0 |
| 4 | Polymer radical (R*) | $[R^*]$ | 0 |
| 5 | Oxygen (O$_2$) | $[O_2]$ | At time (t=0), at top -> Solubility x partial pressure of oxygen; at other -> 0 At bottom always -> $\frac{\partial[O_2]}{\partial y} = 0$ |
| 6 | Per-oxy radical | $[ROO^*]$ | 0 |
| 7 | Alcohol | $[ROH]$ | 0 |
| 8 | Ketone | $[R_2CONH_2]$ | 0 |
| 9 | Carboxylic acid | $[R_2COOH]$ | 0 |
| 10 | Urethane product | $[RO]$ | 0 |

(continued)

| S.No. | Species | Concentration expression | Initial concentrations and boundary conditions |
|---|---|---|---|
| 11 | Crosslinked polymer (R-R) | $[R\text{-}R]$ | 0 |

For all components, the rate equation of production or loss was developed based on the mass balance and rate laws as known in the art. Mass balance for a component is taken as

*Rate of formation or loss = Component Produced - Component Used + Component diffused*

For all the components, only oxygen was the diffusing component, thus component diffused term is non-zero for oxygen mass balance. For other components, diffused term becomes zero automatically.

For oxygen, the mass balance yields the equation is as follows

$$\frac{\partial[O_2]}{\partial t} = \frac{\partial}{\partial y}\left(D_{O_2}\left(\frac{\partial[O_2]}{\partial y}\right)\right) - k_3[R^*][O_2] + k_4[ROO^*]^2$$

For oxygen diffusion the boundary conditions were defined as follows

$$[O_2](t = 0, y) = 0$$

$$[O_2](t, y = 0) = S_{eq} \times P_{O_2}$$

$$\frac{\partial[O_2]}{\partial y}(t, y = l) = 0$$

where, t is the time of simulation, y represents the space dimension (depth of coating), $S_{eq}$ is the solubility of oxygen in polyurethane coating film, $P_{O_2}$ is the partial pressure of the oxygen.

For other components, the mass balance resulted in the following equations with their boundary conditions and initial concentrations.

$$\frac{\partial[RH]}{\partial t} = -k_2[X^*][RH] - k_5[ROO^*][RH] \qquad ; [RH](t = 0, y) = [RH]_0$$

$$\frac{\partial[X-X]}{\partial t} = -k_1\varphi[X-X]E_o e^{-\alpha y[XH]} + k_2[X^*][RH] \quad ; [XH](t = 0, y) = 0.1[RH]_0$$

$$\frac{\partial[X^*]}{\partial t} = 2k_1\varphi[X-X]E_0 e^{-\alpha y[RH]} - k_2[X^*][RH] \qquad ; [X^*](t = 0, y) = 0$$

$$\frac{\partial[R^*]}{\partial t} = k_2[X^*][RH] + k_5[ROO^*][RH] - k_3[R^*][O_2] - k_6[R^*]^2 ; [R^*](t = 0, y) = 0$$

$$\frac{\partial[ROO^*]}{\partial t} = k_3[R^*][O_2] - k_4[ROO^*]^2 - k_5[ROO^*][RH] \qquad ; [ROO^*](t = 0, y) = 0$$

$$\frac{\partial [ROH]}{\partial t} = k_4 [ROO]^2 \qquad ; [ROH](t = 0, y) = 0$$

$$\frac{\partial [RO]}{\partial t} = k_4 [ROO^*]^2 \qquad ; [RO](t = 0, y) = 0$$

$$\frac{\partial [R_1 CONH_2]}{\partial t} = k_5 [ROO^*][RH] \qquad ; [R_1 CONH_2](t = 0, y) = 0$$

$$\frac{\partial [R_2 COOH]}{\partial t} = k_5 [ROO^*][RH] \qquad ; [R_2 COOH](t = 0, y) = 0$$

$$\frac{\partial [R - R]}{\partial t} = 0.5 \, k_6 [R^*]^2 \qquad ; [R - R](t = 0, y) = 0$$

All the above defined equations represent the degradation kinetics mathematically. Out of these reactions, oxygen diffusion is a partial differential equation, while others are ordinary differential equations. To estimate the degradation, all these equations were solved under the set of system parameters and constants, as presented in Table 2. Values for these constants or parameters were taken from literature or separate set of experiments as per the coating chemistry or environmental conditions, while unknown quantities are assumed initially.

Table 2: System parameters for the chemical kinetics mathematical model.

| Parameter with SI Units | Description | Values |
|---|---|---|
| $[RH]_0$ (mol /m3 coating) | Initial concentration of polyurethane | 4082 |
| $P_{O2}$ (Pa) | Partial pressure of oxygen in air (environmental condition) | 21278 |
| $S_{eq}$ (mol /m³ coating Pa) | Solubility of oxygen in polyurethane film | $1.6 \times 10^{-4}$ |
| $D_{O2}$ (m2/s) | Diffusivity of oxygen in polyurethane film | $7.5 \times 10^{-13}$ |
| $\varphi$ (dimensionless) | Quantum yield of urethane resin | $4 \times 10^{-6}$ (assumed) |
| $E_0$ (W/m²) | Radiation intensity of light at coating surface | 30 |
| $\alpha_h$ | Spectral sensitivity of hydrogen abstraction | 1 |
| $k_1$ (m2/W.s) | rate constant for initiation of radical from impurities | 1 (assumed) |
| $k_2$ (m3/mol.s) | rate constant for propagation to urethane linkage | 0.1 (assumed) |
| $k_3$ (m3/mol.s) | rate constant for formation of per-oxy radical | 10 (assumed) |
| $k_4$ (m3/mol.s) | rate constant for termination reaction of per-oxy | 0.1 (assumed) |
| $k_3$ (m3/mol.s) | rate constant for per-oxy reaction with polyurethane | 0.1 (assumed) |
| $k_6$ (m3/mol.s) | rate constant for cross-linking | 1 (assumed) |

The step of iteration by using the first solver and the second solver are better understood by way of following description:

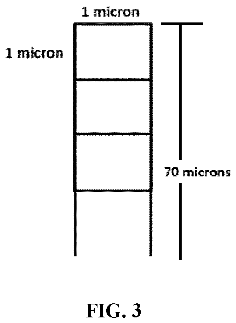

FIG. 3, with reference to FIGS. 1-2, depicts discretization of space along the depth direction in the coating material, in accordance with an embodiment of the present disclosure. The developed chemical kinetics model was solved using numerical techniques. Partial differential equation in the set contains differential terms in the space and time both. Thus, it is discretized into space and time using control volume approach and solved. While ordinary differential equations are solved using the 4th order Runge - Kutta method (RK4). Discretization is done over the coating dimension of 70 μm depth (for optimization, 1D coating is taken) by 1 μm grid as shown FIG. 3. Thus, the length step becomes 1 μm. Discretization in time step is chosen to maintain the stability of the solution, which is taken as around 0.5 s. Time step of solving and total time for which weathering needs to be studied encompass the total simulation time.

Using the approximated system parameters and constants, PDE and ODEs were solved for 80 days of accelerated weathering time in one experimental embodiment of the present disclosure. This time was taken to compare the concentration profiles of components available from experiments. For concentration matching of the reactant polymer and reaction product, experimental data was available in terms of absorbance for the components calculated from the FTIR data. The concentration data from the solution of chemical kinetics model was modified in terms of absorbance by integrating the concentration values over the depth up to which FTIR waves can penetrate. Absorbance can be calculated from the concentration data as follows.

$$A = K \int_{y=0}^{y=70} c(y)\, dy$$

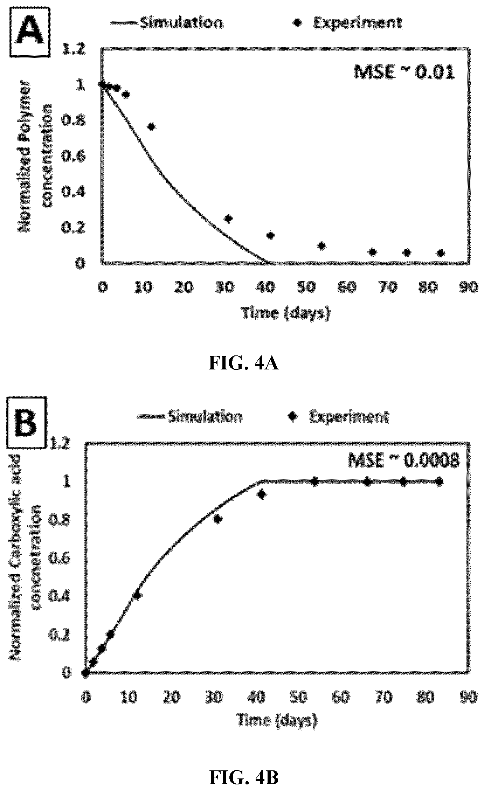

where, $A$ is the absorbance of the component, $K$ is a constant, and $c(y)$ is the concentration of the specie as a function of the depth which was integrated over the depth profiles from 0 $\mu$m (surface) to 70 $\mu$m (depth up to which FTIR wavers penetrate). Absorbance profile and integrated concentration profile of the components (Polymer and carboxylic acid) were normalized with their initial values and were compared to estimate the Mean Square Error (MSE) as shown in FIGS. 4A and 4B. More specifically, FIG. 4A, with reference to FIGS. 1 through 3, depicts a graphical representation illustrating a comparison of polymer normalized concentration from initial solution and experiment, in accordance with an embodiment of the present disclosure. FIG. 4B, with reference to FIGS. 1 through 4A, depicts a graphical representation illustrating a comparison of carboxylic acid normalized concentration from initial solution and experiment, in accordance with an embodiment of the present disclosure.

The concentration profiles of components from the system 100 does not match the experimental concentration profiles as the MSE (0.01 for polymer matching) obtained was extremely high. Two chemical components (polymer and carboxylic acid) were used for this matching. To minimize the error between experimental and kinetics model concentration of components, the system parameters of the listed individual reaction kinetics need to be updated.

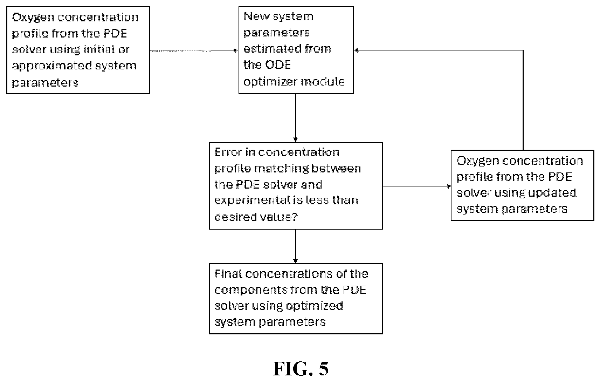

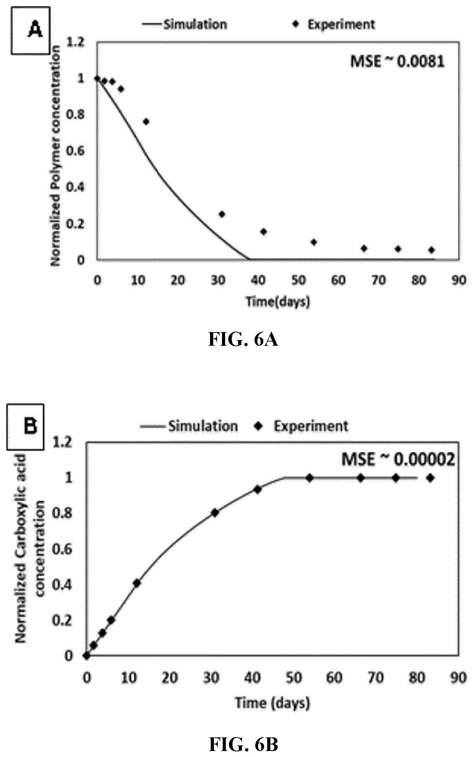

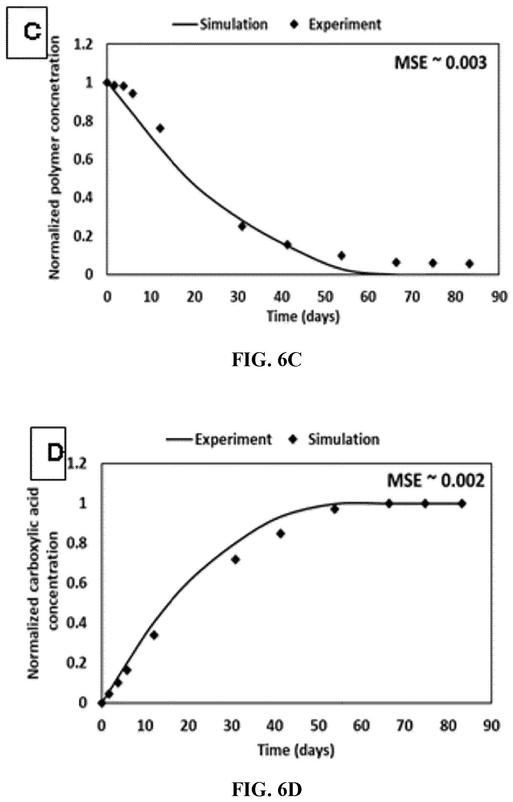

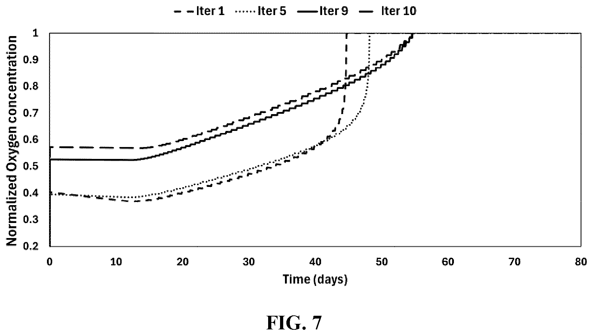

Below description illustrates optimization of the chemical kinetics model based on the optimized set of parameters obtained. For better matching between the experimental and modeled concentrations of components, the system kinetics parameters were estimated using a Particle Swarm Optimization (PSO) algorithm as known in the art. This algorithm finds the set of parameters, for which the error between the concentration of components from model and experiments is low. To conduct fast optimization, the system 100 removed the PDE equation (which was time - taking) from the PSO and oxygen concentrations at various time and space were assumed from the last solution (from approximated system parameters). Thus, the PSO algorithm only used the ODEs, and a stable solution was easily achieved even at higher timesteps too. Both the polymer and carboxylic acid concentrations were taken in the optimization to estimate the best set of system kinetics parameters. Using the PSO algorithm, a new set of system parameters (also referred to as the optimized set of parameters) were estimated and the errors between the concentration of components were calculated. FIG. 5, with reference to FIGS. 1 through FIG. 4B, depicts a flow diagram illustrating the method for estimating the second set of parameters for generating the second model, in accordance with an embodiment of the present disclosure. More specifically, FIG. 5 depicts a cycle of optimization followed to estimate system parameters in accordance with an embodiment of the present disclosure. Further, to achieve better kinetics fitting results, after each set of system parameters were obtained, a new oxygen concentration profile was obtained from the system 100 by solving ODE and PDE in the model. Using the new oxygen concentration profile and rescaling it, optimization was performed again. This cyclic optimization was performed until the MSE of concentration match lies below a certain range. This optimization workflow is shown in FIG. 5 as mentioned above. The concentration profiles obtained during optimization for various iterations (middle and the final iteration) are shown in FIGS. 6A through 6D. After a few iterations, a match in concentrations for both the reactant and product components of the weathering experiment was obtained as the MSE was less than the desired value of 0.005. More specifically, FIGS. 6A through 6D, with reference to FIGS. 1 through 5B, depict graphical representations illustrating concentrations profile matching from model and experiments, wherein FIGS. 6A and 6B depict middle of iterations, and FIGS. 6C and 6D depict from the final iteration, in accordance with an embodiment of the present disclosure. The oxygen concentration profile during the optimization which involved the scaling is shown in FIG. 7. More specifically, FIG. 7, with reference to FIGS. 1 through 6D, depicts a graphical representation illustrating averaged concentration profiles from the first solver (e.g., the PDE solver) during the optimization at different iterations from the second solver (e.g., the ODE solver), in accordance with an embodiment of the present disclosure. An updated list of chemical kinetics reaction parameters from the final iteration (e.g., the optimized set of parameters) is given in Table 3, which shows the deviations of the kinetics parameters from their initial guess values.

Table 3: Optimized system parameters for the chemical kinetics model

| Parameter with SI Units | Description | Values |
|---|---|---|
| $[RH]_0$ (mol /m3 coating) | Initial concentration of polyurethane | 4082 |
| $P_{O2}$ (Pa) | Partial pressure of oxygen in air (environmental condition) | 21278 |
| $S_{eq}$ ($mol\ /m^3\ coating\ Pa$) | Solubility of oxygen in polyurethane film | $1.6 \times 10^{-4}$ |
| $D_{O2}$ ($m^2/s$) | Diffusivity of oxygen in polyurethane film | $7.5 \times 10^{-13}$ |
| $\varphi$ (dimensionless) | Quantum yield of urethane resin | $4.18 \times 10^{-6}$ |
| $E_0$ ($W/m^2$) | Radiation intensity of light at coating surface | 30 |
| $\alpha_h$ | Spectral sensitivity of hydrogen abstraction | 1 |
| $k_1$ ($m^2/W.s$) | rate constant for initiation of radical from impurities | $1.52 \times 10^{-4}$ |
| $k_2$ ($m^3/mol.s$) | rate constant for propagation to urethane linkage | 12.1 |
| $k_3$ ($m^3/mol.s$) | rate constant for formation of per-oxy radical | 1.03 |
| $k_4$ ($m^3/mol.s$) | rate constant for termination reaction of per-oxy | 3.09 |
| $k_3$ ($m^3/mol.s$) | rate constant for per-oxy reaction with polyurethane | 4.42 |
| $k_6$ ($m^3/mol.s$) | rate constant for cross-linking | 2.33 |

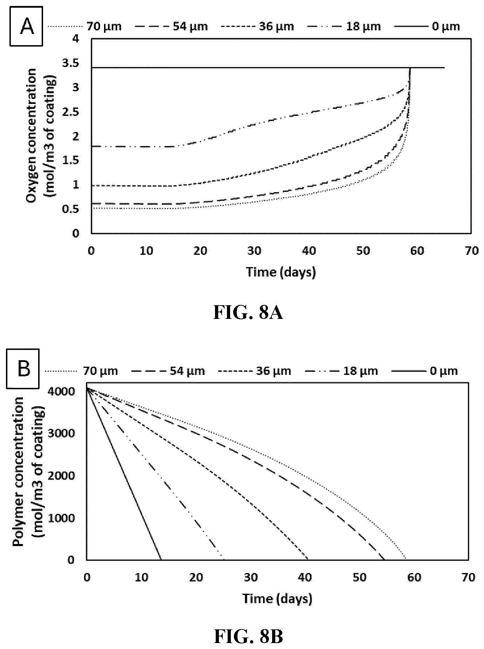

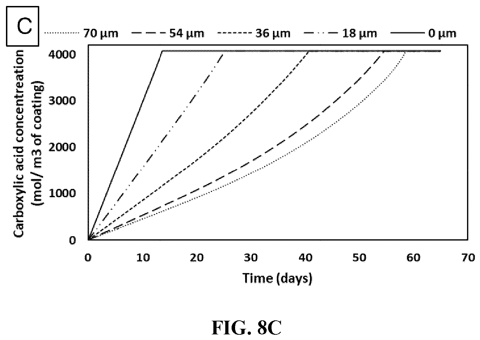

Using the optimized set of kinetics system parameters (e.g., also referred to as the optimized set of parameters), the concentrations of the components were obtained from the model. The concentration profiles with time at different lengths/ depths of the polymer coatings are shown in FIGS. 8A through 8C. More specifically, FIGS. 8A through 8C, with reference to FIGS. 1 through 7, depict graphical representations illustrating concentration profiles of components from the second model using optimized set of kinetics parameters at different depths, in accordance with an embodiment of the present disclosure. FIG. 8A is for oxygen, FIG. 8B is for polymer, and FIG. 8C is for carboxylic acid, respectively. The polymer concentration at the surface decreases faster as compared to the other depths as the UV radiation and oxygen concentration is highest at the surface. Similarly, products such as carboxylic group (figure 6c) generate first at the surface and later at the depths.

Service lifetime of the coating can be calculated by the time the coating has taken to reach 10% concentration of polymer left. At 10% concentration of polymer, the coating is assumed to be ablated. This is in the sense that with degradation, volatile and loose products were produced. These reaction products escape out of the coating and cause the ablation of the coating. Using the optimized set of parameters, the photons being targeted on the coating with time were recorded which helped in setting up the simulation box for physical degradation.

Referring to step 204 of the method of the present disclosure, the system 100 and the method describe generation of the second model (e.g., the physical degradation model) by way of following description. At first a Monte-Carlo (MC) simulation technique is performed on the optimized set of parameters and the plurality of inputs to obtain a set of simulation parameters (e.g., Pixel volume for photodegradation damage obtained from reactive photon count derived from the first model). Using the set of simulation parameters, the system 100 estimates a pixel damage location, and a MC event time series associated with the coating material. To estimate the pixel damage location, and the MC event time series associated with the coating material the system 100 also uses an absorption coefficient of coating polymer. Further, using the pixel damage location, and the MC event time series the system 100 estimate a pore damage time, and a pixel spatiotemporal damage status. A first updated pixel spatiotemporal damage region is then determined based on an associated pit formation and growth identified in the pore damage time, and the pixel spatiotemporal damage status. Furthermore, one or more unconnected islands from the first updated pixel spatiotemporal damage region are filtered to obtain a second updated pixel spatiotemporal damage region. The second updated pixel spatiotemporal damage region serves as the second model (e.g., the physical degradation model). The above steps are further better understood by way of the following description.

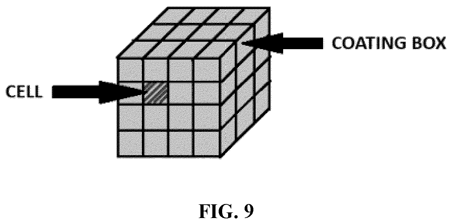

Besides the chemical modification in the coating material, physical modification also occurs. These physical modifications relate to the structural changes in the coating. Characteristics like surface topography, roughness, thickness loss, and mass loss and related mechanical properties etc. are affected by the physical modifications. Service lifetime based on these characteristics also plays a significant role in the application of coatings. These characteristics are predicted by simulating the degradation as the erosion of the surface coating using a Monte Carlo model setup as depicted in FIG. 9. More specifically, FIG. 9, with reference to FIGS. 1 through 8C, depicts the coating material as simulation box of specific depth divided into cells of specific dimension (e.g., 1-micron dimension), in accordance with an embodiment of the present disclosure.

In the Monte Carlo model, the polymer is degraded randomly using ablating photons from the UV radiation. Models generate spatiotemporal changes in the surface topography and chemistry. Using these new topography and chemistry, statistical properties like RMS roughness, roughness profiles, mass and thickness loss are calculated. These properties can be extended to calculate the real time dependent properties such as gloss loss, fracture strength, wetting angle, etc. The step of performing the Monte-Carlo (MC) simulation technique on the optimized set of parameters and the plurality of inputs to obtain the set of simulation parameters is described below.

The Monte Carlo method is a technique to evolve a system based on the probabilistic random repeated events. Repeated events are the degradation of the polymer by the ablating photon with an assignable probability of absorption. Ablating photons are the photons which can degrade the polymer component if it is absorbed. The second model (e.g., physical degradation model) consists of a coating layer (0-70 $\mu$m (depth) x 20 $\mu$m (width) x 1 $\mu$m (length)) divided into small boxes or cells or pixels as shown in FIG. 9 by way of example. Within any one grid is sufficient for polymer component to be removed by the ablating photon targeted event. Grid dimensions were calculated based on the photon count from the chemical kinetics model (e.g., the first model).

For polyurethane system, the average depth of absorption of a photon from UV A radiation has been 6.36 $\mu$m. Within this depth, 61% of the targeted photons were absorbed by the coating material. From chemical kinetics model, number of photons utilized to ablate a 6$\mu$m depth coating were calculated. Using this count, the volume being ablated by one single ablating photon was calculated to be 47.5 nm x 47.5 nm x 47.5 nm. This volume contained sufficient material to be ablated when an ablating photon reaction event occurs and termed as pixel volume for coating damage for further references in this study. For each pixel volume, there was a probability assigned as to whether photon was absorbed or not. This probability was based on the UV ray absorption coefficient of the material and depth.

Referring to the step of obtaining the second model, the system 100 describes estimation of the pixel damage location, and the MC event time series from the set of simulation parameters as below:

In an ablating photon reaction event, a photon was targeted on the coating box. The location of the photon at which cell it is targeted was chosen randomly by the model. With each photon, the model generated a random number. If the random number was less than the assigned probability for the material in the targeted cell, the cell was assumed to be ablated and form volatile products. This probability of degradation is termed as the quantum efficiency of the polymer to get degraded by the UV radiation. Every successful and unsuccessful event to ablate material was recorded to identify the time of the degradation events which depict the pixel damage location, and the MC event time series.

Further, the step of estimating a pore damage time, and a pixel spatiotemporal damage status using the pixel damage location, and the MC event time series is better understood by way of following description:

With degradation events, the status of the polymeric material in the cells was represented by numbers. If the cell contains undegraded polymer, then it was represented by '1'. If the cell contains degraded/ablated polymer, then the status was represented by '2'. This pixel status for various locations was recorded with time and termed as 'spatiotemporal damage status' for further references and analysis by the system 100 and the method of the present disclosure.

After every successful degradation event, pixel spatiotemporal damage status was updated to obtain the first updated pixel spatiotemporal damage region. Based on the location of the degradation event, cells with degraded polymers were separated. The cells which were degraded on the surface of the coating escape out and form a vacuum. While the other type of damaged pixels diffuses out when they meet the vacuum. Such vacuum cells were termed as 'pits' while others in bulk were termed as 'pore'. For pit formation, the pixel spatiotemporal damage status was changed to '0' from '2'. For pore formation, damage time was recorded to define the age of the degradation event, while the pixel temporal damage status was not updated.

Pores were allowed to diffuse out only after they encounter pits with a 4-way connectivity, and then the pores were converted to pits. Pores diffuse according to their age. The pores which were generated first diffuse out first compared to the pores formed later, even if all were in contact with same pit at same time. This allows the second model to mimic the real diffusion process effectively. As these pores diffuse out, they leave the vacuum at their location and thus the pit grows to the pore location. Thus, the pixel spatiotemporal damage status was updated accordingly along with the time of pore damage (e.g., also referred to as the first updated pixel spatiotemporal damage region).

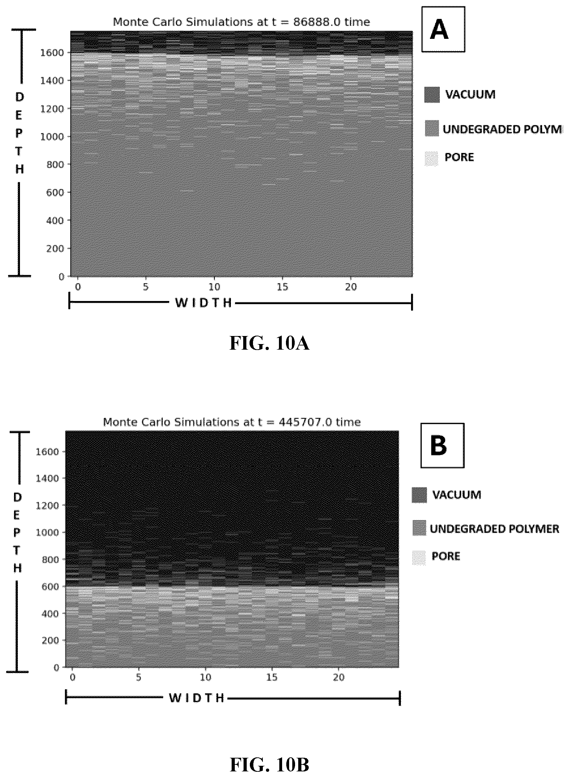

The pixel spatiotemporal damage status defines the coating chemistry (degraded or not) at any time and using this, surface topography along the depth of the material was evaluated as shown in FIGS. 10A and 10B. More specifically, FIGS. 10A and 10B, with reference to FIGS. 1 through 9, depict surface topography along the depth of the coating by the Monte Carlo simulations at MC time of degraded events (e.g., 86888 events in FIG. 10A, 445707 events in FIG. 10B), in accordance with an embodiment of the present disclosure.

Referring to steps of generating the second model, the step of filtering one or more unconnected islands from the first updated pixel spatiotemporal damage region to obtain a second updated pixel spatiotemporal damage region is described below:

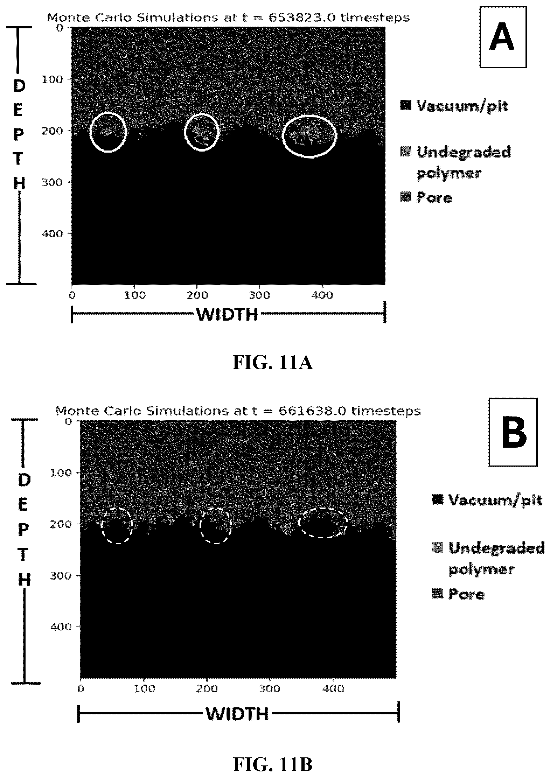

During degradation, islands of pores or undegraded polymer were formed which were surrounded by the vacuum and disconnected from the bulk of the coating material. Such islands are termed as unconnected islands and are lost from the coating material during real weathering testing due to rain or wind. The system 100 is configured by the instructions to identify and remove such unconnected islands. This makes the system 100 mimic the weathering to a closer practical sense. These unconnected islands were formed after a certain degradation event had occurred. So, conventional methods such as image processing tools like OpenCV in python were implemented by the system 100 identify and remove/filter these unconnected islands. The connected component method of OpenCV scanned through the whole coating MC simulation box and segregated out the unconnected islands. The connected component utilized the 4-way connectivity check in the pixel spatiotemporal damage status (including the first updated pixel spatiotemporal damage region) and identified the disconnected components. As these unconnected islands were removed the status of the cells were changed to vacuum and the pixel spatiotemporal damage status was updated (e.g., the second updated pixel spatiotemporal damage region). FIGS. 11A and 11B, with reference to FIGS. 1 through 10B, show the surface topography at two timesteps of subsequent successful coating degradation (of dimensions $20\mu m$ x $20\mu m$ x $40nm$) in which vacuum/pit is represented by the black color, undegraded polymer is represented by light grey color, pore is represented by dark grey color, in accordance with an embodiment of the present disclosure. Unconnected islands of pore and polymer are encircled (solid in FIG. 11A and dotted in FIG. 11B). These islands marked in FIG. 11A were removed and converted into vacuum in the FIG. 11B. The second updated pixel spatiotemporal damage region therein obtained serves as the second model.

Referring to steps of FIG. 2, at step 206 of the method of the present disclosure, the one or more hardware processors 104 combine the first model (e.g., the chemical kinetics model) and the second model (e.g., the physical degradation model) to obtain a combined model. More specifically, in the present disclosure, the combined model is obtained or generated by correlating each of the one or more polymer concentration profiles of the first model with one or more timesteps of the second updated pixel spatiotemporal damage region serving as the second model. The combined model comprises the one or more polymer concentration profiles from the first model, and a time scaling factor and the second updated pixel spatiotemporal damage region serving as the second model, in one embodiment of the present disclosure. The step of combining the first model and the second model to obtain the combined model is better understood by way of following description:

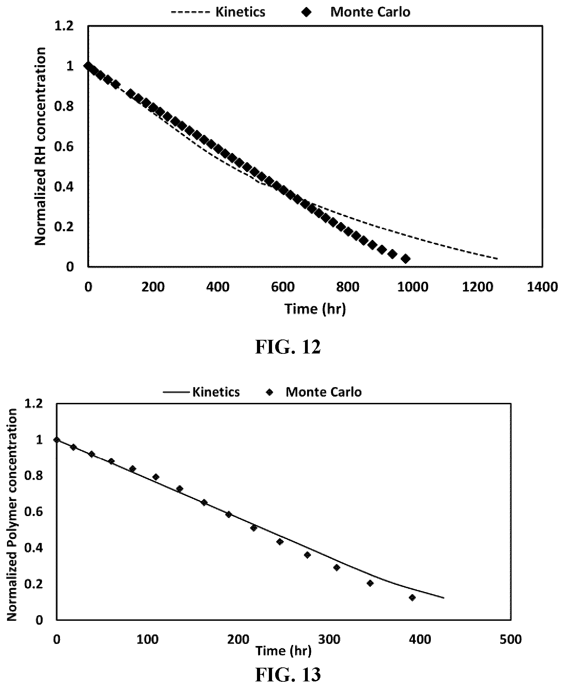

Surface topography of the coating material was obtained using the pixel spatiotemporal damage status (e.g., the second updated pixel spatiotemporal damage region) successfully. For estimation of the service lifetime, the Monte Carlo timestep needs to be related to the real experimental weathering time. To scale the MC timestep, the concentration profile of the polymer from the Monte Carlo model was correlated to the concentration profile of the polymer from the experiments or chemical kinetics model. Time scaling was done by finding an average ratio between the Monte Carlo timestep and chemical kinetics model's time at similar polymer concentrations. For Monte Carlo simulations in which the unconnected islands are not removed, the timestep of Monte Carlo was ~ 5.39 s of chemical kinetics model. While in case of a 20 $\mu m$ depth coating taken for simulation and unconnected islands were removed, the timestep of Monte Carlo was ~ 1.77s of chemical kinetics model. Normalized concentration profiles of polymer in coating with scaled time is shown in FIGS. 12 and 13 respectively. More specifically, FIG. 12, with reference to FIGS. 1 through 11B, depicts a graphical representation illustrating a comparison of a normalized RH (polymer) concentration between chemical kinetics model and physical degradation model for a 70-micron coating, in accordance with an embodiment of the present disclosure. In Monte Carlo simulation, the unconnected islands were not removed. FIG. 13, with reference to FIGS. 1 through 12, depicts a graphical representation illustrating a comparison of the normalized RH (polymer) concentration between chemical kinetics model and physical degradation model for a 20-micron depth coating, in accordance with an embodiment of the present disclosure. In this Monte Carlo simulation, the unconnected islands were removed. This scaling built a successful relationship between the chemical kinetics model and physical degradation model and results can be inferred from each other. Using the time scaling factor and pixel spatiotemporal damage status, surfaces properties in real time can be (or are) calculated.

Once the combined model is obtained by combining the first model and the second model, at step 208 of the method of the present disclosure, the one or more hardware processors 104 estimate the service lifetime, and the degradation of the coating material. First a surface profile of the coating material is developed using the pixel spatiotemporal damage status associated with the second model. Further, a thickness loss and a roughness parameter of the coating material are calculated based on the surface profile. Then, a gloss loss, a fracture toughness and a contact wetting angle of the coating material are calculated using the thickness loss and the roughness parameter. Finally, the service lifetime, and the degradation of the coating material are estimated based on the gloss loss, the fracture toughness, and the contact wetting angle using the combined model.

The step of estimating the service lifetime, and the degradation of the coating material using the combined model may be further better understood by way of following description:

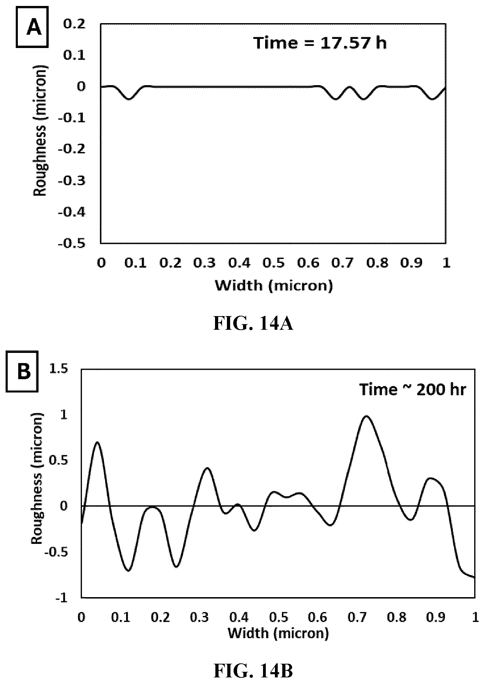

As the coating material degrades, the thickness starts to decrease due to the loss of polymer from the surface. This polymer loss is not uniform throughout the surface and results in roughening of the surface. From the Monte Carlo simulation, the pixel spatiotemporal damage status/region (e.g., the first and the second updated pixel spatiotemporal damage region) generated the status in change of polymer concentration at the surface up to 40 nm resolutions. Scans of varied sizes (40nm x 40nm or 200nm x 200nm or 1000nm x 1000nm) were made and depth of coating over those scans were obtained. Using the depth on these scans, the surface profiles are generated as shown in FIGS. 14A through 14D.

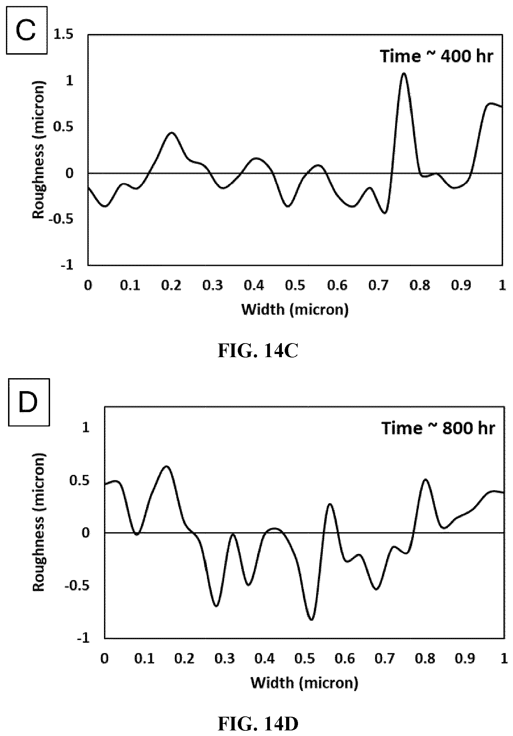

More specifically, FIGS. 14A through 14D, with reference to FIGS. 1 through 13, depict graphical representations illustrating surface roughness profiles developed from the pixel temporal damage status/region (e.g., the first and second updated pixel spatiotemporal damage region) of a 70-micron depth coating at various times of 17.57 h, 200 h, 400 h, and 800 h, respectively, in accordance with an embodiment of the present disclosure. For instance, FIG. 14A depicts surface roughness profiles developed from the pixel temporal damage status/region (e.g., the first and second updated pixel spatiotemporal damage region) of a 70-micron depth coating at time of 17.57 h. FIG. 14B surface roughness profiles developed from the pixel temporal damage status/region (e.g., the first and second updated pixel spatiotemporal damage region) of a 70-micron depth coating at various times of 200 h. FIG. 14C surface roughness profiles developed from the pixel temporal damage status/region (e.g., the first and second updated pixel spatiotemporal damage region) of a 70-micron depth coating at various times of 400 h. FIG. 14D surface roughness profiles developed from the pixel temporal damage status/region (e.g., the first and second updated pixel spatiotemporal damage region) of a 70-micron depth coating at various times of 800 h.

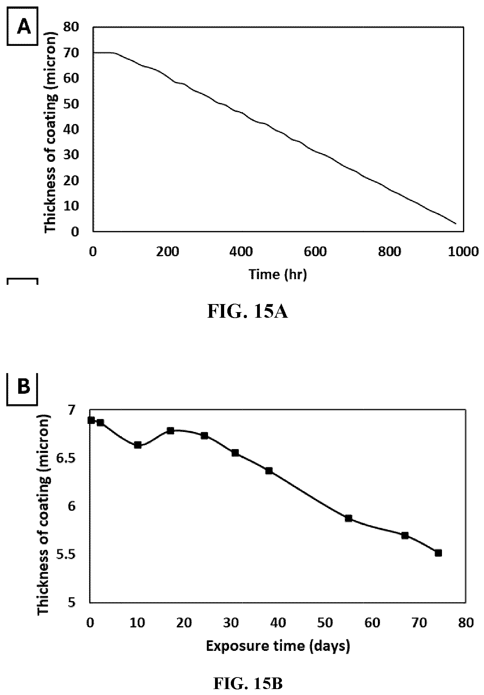

From the pixel temporal damage status (the first and second updated pixel spatiotemporal damage region), the concentration of the polymer remaining in the coating material was obtained. Using the concentration profile, thickness loss was calculated by depth up to which the polymer has been vacuumized. Thickness loss for a 70-micron depth coating sample/material with respect to real accelerated weathering time is shown in FIG. 15A. For an epoxy based polyurethane coating degrading under natural outdoor exposure shows a similar trend in decrement of the thickness change of the coating material is observed as shown in FIG. 15B. More specifically, FIGS. 15A and 15B, with reference to FIGS. 1 through 14D, depict graphical representations illustrating thickness of the coating varying with time depicting the loss with real accelerated weathering time from physical model (e.g., refer FIG. 15A), and with natural outdoor exposure of epoxy-based PU coating (e.g., refer FIG. 15B), in accordance with an embodiment of the present disclosure.

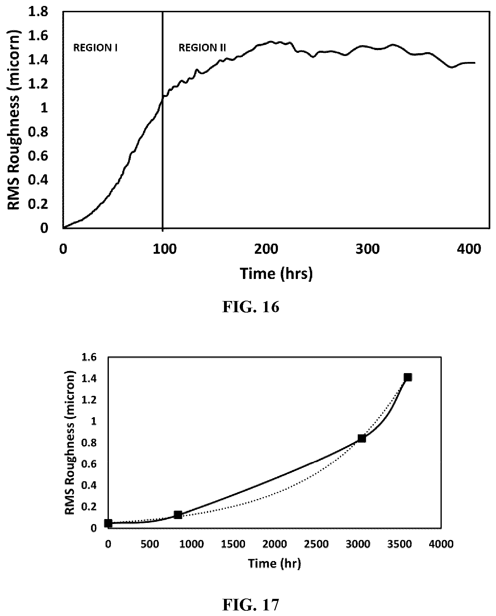

Using the above obtained surface profiles, RMS roughness was calculated by the standard deviation of the roughness profile data. RMS roughness for the 70-micron coating is shown in FIG. 16. RMS roughness can be divided into two different regions according to their characteristics. Region I in FIG. 16 shows an exponential increase in RMS roughness during initial exposure. This exponential increment trend has been observed in experimental 1 for similar types of coating under different exposure as shown in FIG. 17. Region II marks the saturation of RMS roughness by following a parabolical trajectory in FIG. 16. This trend is observed after certain time of exposure has been carried. This trend is also observed in FIG. 17 in the ending times of exposure. However, this trend matching for RMS roughness is carried out in FIG. 17 is for acrylic based polyurethane coating as exact RMS roughness plots for the same polymer coating under similar exposures of degradation were not available. Therefore, to quantify the combined model, the range values of RMS roughness have also been matched with the available literature results.

For acrylic based polyurethane coating, RMS roughness values under natural outdoor exposure has been reported to increase to 0.987 $\mu$m from 0.199 $\mu$m. In another experimental for natural exposure the RMS roughness has been reported to be 1.39 $\mu$m after 5 years. RMS roughness for coating under accelerated degradation from our model predicts the variation in same range (0 - 1.5 $\mu$m).

Other roughness parameters like $R_a$ (*average roughness*), $R_z$ and $R_t$ can also be calculated. Average roughness for acrylic based polyurethane coating has been reported to vary in the range of 0.134 $\mu$m to 0.580 $\mu$m under natural exposure. Similar range (0 to 0.35 $\mu$m) of average roughness has been obtained from the physical degradation model. FIG. 16, with reference to FIGS. 1 through 15B, depicts a graphical representation illustrating a root mean square (RMS) Roughness of the 70-micron depth coating with accelerated weathering degradation time evaluated from the Monte Carlo simulations, in accordance with an embodiment of the present disclosure. FIG. 17, with reference to FIGS. 1 through 16, depicts a graphical representation illustrating a root mean square (RMS) Roughness of the acrylic based polyurethane coating from weathering under different accelerated exposure conditions, in accordance with an embodiment of the present disclosure. These qualitatively verify the RMS roughness obtained from the physical gradation model of coating degradation.

A failure criterion for the coating material may be crack propagation through the coating material ultimately causing channeling cracks, delamination, etc. By applying the Griffith crack criterion and using the maximum flaw size generated, the relative strength of the coating material with time can be estimated. Maximum flaw size in the surface of coating was calculated from the surface roughness profiles. By Griffith criterion,

$$\sigma_G = \sqrt{\left(\frac{2E\gamma_s}{\pi\alpha}\right)}$$

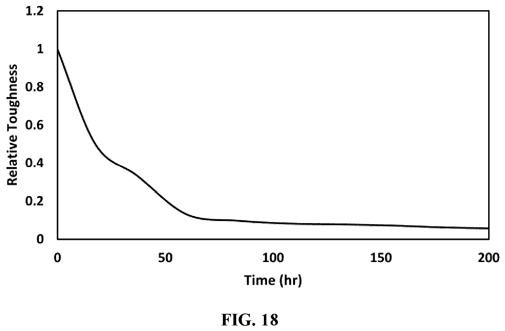

where, $\sigma_G$ is the critical stress at which crack propagates, $E$ is the Young's modulus, $\gamma_s$ is the surface energy per unit area, and $\alpha$ is the maximum flaw size. The decrease in fracture toughness was represented by the relative fracture toughness as compared to the initial fracture toughness (before weathering) as shown in FIG. 18. FIG. 18, with reference to FIGS. 1 through 17, depicts a graphical representation illustrating a change in relative fracture toughness of the coating material with time of degradation, in accordance with an embodiment of the present disclosure.

The following relations can obtain estimates of other surface macroscopic properties.

Wetting contact angle changes with the roughness of the coating surface. Change in contact angle is a modified version of Young's modulus, which incorporates the ratio of actual surface area to the projected surface. Thus, wetting of a surface is a function of the physical profile of the surface and the surface chemistry. It is calculated as

$$\theta = \arccos\left(\frac{r\left(\gamma_{solid-vapor} - \gamma_{solid-liquid}\right)}{\gamma_{liquid-vapor}}\right)$$

where, $\theta$ is the contact angle, $r$ is the ratio of actual to projected area of solid contact with the solid phase. The $\gamma$ are the surface free energy between the subscripted surfaces.

The gloss can be predicted by the specular reflectance at normal incidence ($R_s$), given as

$$R_s = R_0 e^{-\left(\frac{4\pi\sigma\cos(\psi)}{\lambda}\right)^2}$$

where, $R_0$ is the reflectance of the perfectly smooth surface of the same material, $\lambda$ is the wavelength of the incident light, $\sigma$ is the standard deviation of the surface from its mean level, and \psi is the specular angle.

Gloss loss calculated by the loss in the reflectance of the material can be used to predict the service life of the paint when it falls below a critical value (e.g., say a threshold $m$ having some value - $p$).

The system 100 then estimates the service lifetime, and the degradation of the coating material based on the gloss loss, the fracture toughness, and the contact wetting angle and this estimation is depicted by way of the following. Service life of the coating material can be calculated by the estimation of the these above calculated properties/parameters as:

*Service life = Total life of coating as per the critical value of property - Actual life of coating passed as per property current value*

Embodiments of the present disclosure described a working example of the system 100 for an automotive based coating under a defined accelerated weathering exposure condition. The chemical kinetics model based on the degradation mechanism of the coating material is mentioned and optimization procedures for the model are described herein. Chemical modification in terms of concentration profiles of the components is shown in the case-study above. Based on the chemical model, physical degradation model is developed, and simulation techniques procedure is also described above. Real time physical changes in terms of concentration plot, and surface topography are obtained. The variation in surface properties like surface roughness, thickness loss, gloss loss, fracture toughness during the degradation from the physical changes are calculated that enable the system to determine the service lifetime and degradation of the coating material.

1. Efficient chemical model: To solve the equations involved in the degradation of the coatings, system parameters were to be optimized for an efficient model. An iterative particle swarm optimization was used to estimate the parameters by minimizing the errors in concentrations of the components (reactant and product in degradation) from the experimental data. Some equation solvers made the optimization time consuming. Therefore, phase wise optimization in which slow equation solvers were removed, was carried out to obtain system parameters efficiently and fast. Using optimized system parameters, the model estimated correct chemical changes in coating, which were validated with experimental data.

2. Diffusion of the trapped products: All the volatile products formed during the degradation tend to escape out of the coating. The products which are formed in the bulk of the coating remain trapped until they meet the surface. While the products formed on the surface escape out and pit is developed in the coating. The trapped products encounter these pits and escape out by diffusion. This diffusion is added in physical model by incorporating the escape or removal only after a sufficient length contact with pit is made and that too as per their age of degradation. Degraded products which have degraded earlier will diffuse out first.

3. Computer vision-based removal of unsupported products: During degradation, clusters of unsupported islands are formed which are lost due to rain or wind in experimental testing. In physical models, such unsupported clusters of components are identified using a computer vision tool. This computer vision tool segregates the connected components in coating, which are undegraded polymer, degraded polymer (both trapped and escaped out). Out of these components, the trapped polymer and undegraded polymer which are in contact with or surrounded by the escaped-out component are termed as unsupported and removed after certain degradation events.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method, comprising:

   receiving, via one or more hardware processors, a plurality of inputs pertaining to a coating material comprising a thickness, a polymer chemistry, a sunlight-Ultraviolet (UV) time series, an oxygen concentration, a moisture concentration, one or more polymer concentration profiles, and a product concentration of polymers (202);
   generating, via the one or more hardware processors, (i) a first model and a second model based on the plurality of inputs (204), wherein the second model is further based on one or more oxygen concentration profiles and number of reactive photons obtained from the first model;

combining, via the one or more hardware processors, the first model and the second model to obtain a combined model (206); and

estimating, via the one or more hardware processors, a service lifetime, and a degradation of the coating material using the combined model (208).

2. The processor implemented method as claimed in claim 1, wherein the first model is obtained by:

generating a first set of parameters using the plurality of inputs;

iteratively performing:

applying, by using a first solver, a first set of governing equations on the first set of parameters to obtain a second set of parameters; and

applying, by using a second solver, a second set of governing equations on the second set of parameters, until an optimized set of parameters is obtained; and

generating the first model using the optimized set of parameters.

3. The processor implemented method as claimed in claim 2, wherein the second model is obtained by:

performing a Monte-Carlo (MC) simulation technique on the optimized set of parameters and the plurality of inputs to obtain a set of simulation parameters;

estimating a pixel damage location, and a MC event time series from the set of simulation parameters;

estimating a pore damage time, and a pixel spatiotemporal damage status using the pixel damage location, and the MC event time series;

obtaining a first updated pixel spatiotemporal damage region based on an associated pit formation and growth identified in the pore damage time, and the pixel spatiotemporal damage status; and

filtering one or more unconnected islands from the first updated pixel spatiotemporal damage region to obtain a second updated pixel spatiotemporal damage region, wherein the second updated pixel spatiotemporal damage region serves as the second model.

4. The processor implemented method as claimed in claim 3, wherein the combined model is obtained by correlating each of the one or more polymer concentration profiles of the first model with one or more timesteps of the second updated pixel spatiotemporal damage region serving as the second model to obtain the combined model, and wherein the combined model comprises the one or more polymer concentration profiles from the first model, and a time scaling factor and the second updated pixel spatiotemporal damage region serving as the second model.

5. The processor implemented method as claimed in claim 1, wherein the step of estimating the service lifetime, and the degradation of the coating material using the combined model comprises:

developing a surface profile of the coating material using the status associated with the second model;

calculating a thickness loss and a roughness parameter of the coating material based on the surface profile;

calculating a gloss loss, a fracture toughness and a contact wetting angle of the coating material using the thickness loss and the roughness parameter; and

estimating the service lifetime, and the degradation of the coating material based on the gloss loss, the fracture toughness, and the contact wetting angle.

6. A system (100), comprising:

a memory (102) storing instructions;

one or more communication interfaces (106); and

one or more hardware processors (104) coupled to the memory (102) via the one or more communication interfaces (106), wherein the one or more hardware processors (104) are configured by the instructions to:

receive a plurality of inputs pertaining to a coating material comprising a thickness, a polymer chemistry, a sunlight-Ultraviolet (UV) time series, an oxygen concentration, a moisture concentration, one or more polymer concentration profiles, and a product concentration of polymers;

generate (i) a first model and a second model based on the plurality of inputs, wherein the second model is further based on one or more oxygen concentration profiles and number of reactive photons obtained from the first model;
combine the first model and the second model to obtain a combined model; and
estimate a service lifetime, and a degradation of the coating material using the combined model.

7. The system as claimed in claim 6, wherein the first model is obtained by:

generating a first set of parameters using the plurality of inputs;
iteratively performing:

applying, by using a first solver, a first set of governing equations on the first set of parameters to obtain a second set of parameters; and
applying, by using a second solver, a second set of governing equations on the second set of parameters, until an optimized set of parameters is obtained; and

generating the first model using the optimized set of parameters.

8. The system as claimed in claim 7, wherein the second model is obtained by:

performing a Monte-Carlo (MC) simulation technique on the optimized set of parameters and the plurality of inputs to obtain a set of simulation parameters;
estimating a pixel damage location, and a MC event time series from the set of simulation parameters;
estimating a pore damage time, and a pixel spatiotemporal damage status using the pixel damage location, and the MC event time series;
obtaining a first updated pixel spatiotemporal damage region based on an associated pit formation and growth identified in the pore damage time, and the pixel spatiotemporal damage status; and
filtering one or more unconnected islands from the first updated pixel spatiotemporal damage region to obtain a second updated pixel spatiotemporal damage region, wherein the second updated pixel spatiotemporal damage region serves as the second model.

9. The system as claimed in claim 8, wherein the combined model is obtained by correlating each of the one or more polymer concentration profiles of the first model with one or more timesteps of the second updated pixel spatiotemporal damage region serving as the second model to obtain the combined model, and wherein the combined model comprises the one or more polymer concentration profiles from the first model, and a time scaling factor and the second updated pixel spatiotemporal damage region serving as the second model.

10. The system as claimed in claim 6, wherein the service lifetime, and the degradation of the coating material are estimated using the combined model by

developing a surface profile of the coating material using the pixel spatiotemporal damage status associated with the second model;
calculating a thickness loss and a roughness parameter of the coating material based on the surface profile;
calculating a gloss loss, a fracture toughness and a contact wetting angle of the coating material using the thickness loss and the roughness parameter; and
estimating the service lifetime, and the degradation of the coating material based on the gloss loss, the fracture toughness, and the contact wetting angle.

11. One or more non-transitory machine-readable information storage mediums comprising one or more instructions which when executed by one or more hardware processors cause:

receiving a plurality of inputs pertaining to a coating material comprising a thickness, a polymer chemistry, a sunlight-Ultraviolet (UV) time series, an oxygen concentration, a moisture concentration, one or more polymer concentration profiles, and a product concentration of polymers;
generating (i) a first model and a second model based on the plurality of inputs, wherein the second model is further based on one or more oxygen concentration profiles and number of reactive photons obtained from the first model;
combining the first model and the second model to obtain a combined model; and
estimating a service lifetime, and a degradation of the coating material using the combined model .

12. The one or more non-transitory machine-readable information storage mediums as claimed in claim 11, wherein the first model is obtained by:

   generating a first set of parameters using the plurality of inputs;
   iteratively performing:

   applying, by using a first solver, a first set of governing equations on the first set of parameters to obtain a second set of parameters; and
   applying, by using a second solver, a second set of governing equations on the second set of parameters, until an optimized set of parameters is obtained; and

   generating the first model using the optimized set of parameters.

13. The one or more non-transitory machine-readable information storage mediums as claimed in claim 12, wherein the second model is obtained by:

   performing a Monte-Carlo (MC) simulation technique on the optimized set of parameters and the plurality of inputs to obtain a set of simulation parameters;
   estimating a pixel damage location, and a MC event time series from the set of simulation parameters;
   estimating a pore damage time, and a pixel spatiotemporal damage status using the pixel damage location, and the MC event time series;
   obtaining a first updated pixel spatiotemporal damage region based on an associated pit formation and growth identified in the pore damage time, and the pixel spatiotemporal damage status; and
   filtering one or more unconnected islands from the first updated pixel spatiotemporal damage region to obtain a second updated pixel spatiotemporal damage region, wherein the second updated pixel spatiotemporal damage region serves as the second model.

14. The one or more non-transitory machine-readable information storage mediums as claimed in claim 13, wherein the combined model is obtained by correlating each of the one or more polymer concentration profiles of the first model with one or more timesteps of the second updated pixel spatiotemporal damage region serving as the second model to obtain the combined model, and wherein the combined model comprises the one or more polymer concentration profiles from the first model, and a time scaling factor and the second updated pixel spatiotemporal damage region serving as the second model.

15. The one or more non-transitory machine-readable information storage mediums as claimed in claim 11, wherein the step of estimating the service lifetime, and the degradation of the coating material using the combined model comprises:

   developing a surface profile of the coating material using the status associated with the second model;
   calculating a thickness loss and a roughness parameter of the coating material based on the surface profile;
   calculating a gloss loss, a fracture toughness and a contact wetting angle of the coating material using the thickness loss and the roughness parameter; and
   estimating the service lifetime, and the degradation of the coating material based on the gloss loss, the fracture toughness, and the contact wetting angle.

SYSTEM
100

MEMORY
102

DATABASE
108

HARDWARE PROCESSOR(S)
104

INTERFACE(S)
106

**FIG. 1**

receiving, via one or more hardware processors, a plurality of inputs pertaining to a coating material comprising a thickness, a polymer chemistry, a sunlight-Ultraviolet (UV) time series, an oxygen concentration, a moisture concentration, one or more polymer concentration profiles, and a product concentration of polymers — 202

generating, via the one or more hardware processors, (i) a first model and a second model based on the plurality of inputs, wherein the second model is further based on one or more oxygen concentration profiles and number of reactive photons obtained from the first model — 204

combining, via the one or more hardware processors, the first model and the second model to obtain a combined model — 206

estimating, via the one or more hardware processors, a service lifetime, and a degradation of the coating material using the combined model — 208

**FIG. 2**

**FIG. 3**

**FIG. 4A**

**FIG. 4B**

Oxygen concentration profile from the PDE solver using initial or approximated system parameters

New system parameters estimated from the ODE optimizer module

Error in concentration profile matching between the PDE solver and experimental is less than desired value?

Oxygen concentration profile from the PDE solver using updated system parameters

Final concentrations of the components from the PDE solver using optimized system parameters

**FIG. 5**

**FIG. 6A**

**FIG. 6B**

**FIG. 6C**

**FIG. 6D**

**FIG. 7**

FIG. 8A

FIG. 8B

FIG. 8C

CELL

COATING BOX

**FIG. 9**

FIG. 10A

FIG. 10B

FIG. 11A

FIG. 11B

**FIG. 12**

**FIG. 13**

FIG. 14A

FIG. 14B

**FIG. 14C**

**FIG. 14D**

**FIG. 15A**

**FIG. 15B**

**FIG. 16**

**FIG. 17**

**FIG. 18**

Europäisches
Patentamt
European
Patent Office
Office européen
des brevets

# EUROPEAN SEARCH REPORT

**Application Number**

EP 25 18 4145

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | AMBESH GUPTA ET AL: "Combined concurrent Physical and Chemical model for accelerated weathering damages of polyurethane-based coatings", ARXIV.ORG, CORNELL UNIVERSITY LIBRARY, 201 OLIN LIBRARY CORNELL UNIVERSITY ITHACA, NY 14853, 16 October 2024 (2024-10-16), XP091918812, * the whole document * | 1-15 | INV. G16C60/00 ADD. C09D175/04 |
| X | EP 4 369 347 A1 (TATA CONSULTANCY SERVICES LTD [IN]) 15 May 2024 (2024-05-15) | 1-3,5-8, 10-13 | |
| A | * the whole document * | 4,9,14 | |
| X | ADEMA KOEN N. ET AL: "Kinetic Monte Carlo simulation of the photodegradation process of polyester-urethane coatings", PHYSICAL CHEMISTRY CHEMICAL PHYSICS, vol. 17, no. 30, 7 July 2015 (2015-07-07), pages 19962-19976, XP093324663, ISSN: 1463-9076, DOI: 10.1039/C5CP01581B | 1-3,5-8, 10-13 | |
| A | * the whole document * | 4,9,14 | |
| A | EP 4 336 163 A1 (TATA CONSULTANCY SERVICES LTD [IN]) 13 March 2024 (2024-03-13) * the whole document * | 1-15 | |

**TECHNICAL FIELDS SEARCHED (IPC)**

G16C
G01N
G06F
C09D

The present search report has been drawn up for all claims

| Place of search | Date of completion of the search | Examiner |
|---|---|---|
| The Hague | 14 October 2025 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 4145

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2025

| Patent document cited in search report | | Publication date | Patent family member(s) | | Publication date |
|---|---|---|---|---|---|
| EP 4369347 | A1 | 15-05-2024 | EP | 4369347 A1 | 15-05-2024 |
| | | | US | 2024177811 A1 | 30-05-2024 |
| EP 4336163 | A1 | 13-03-2024 | EP | 4336163 A1 | 13-03-2024 |
| | | | US | 2024086585 A1 | 14-03-2024 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421048797